# EUROPEAN PATENT SPECIFICATION

(11) **EP 1 927 315 B1**
(45) Date of publication and mention of the grant of the patent: **08.12.2010**
(21) Application number: 07019604.3
(22) Date of filing: 08.10.2007
(51) Int. Cl.: A61B 3/16

(54) **Method for measuring intraocular pressure**
Verfahren zur Messung des Intraokulardrucks
Procédé de mesure de pression intraoculaire

(30) Priority: 30.11.2006 CN 200610172996
(43) Date of publication of application: 04.06.2008
(73) Proprietor: ICARE FINLAND OY, 02600 ESPOO (FI)
(72) Inventor: Kontiola, Antti, 00660 Helsinki (FI)
(74) Representative: Söderman, Päivi Karin Lisbeth

(56) References cited:
- DE-A1-102004 001 675
- US-A- 6 093 147
- US-A1- 2005 137 474

## Description

### TECHNICAL FIELD

The present invention relates to a method for measuring intraocular pressure.

### BACKGROUND ART

Intraocular pressure (IOP) is the fluid pressure inside the eye. It may become elevated due to anatomical problems, inflammation of the eye, genetic factors, as a side-effect from medication, or during exercise. If the IOP is elevated, it can cause pressure within the eye to increase and damage the optic nerve. Since abnormal pressures usually don't cause symptoms, it's very important to have the pressure checked regularly.

The intraocular pressure is generally measured with a special instrument called a tonometer, which is placed on the surface of the cornea measuring its elasticity using various methods (Goldmann tonometer, Schiötz tonometer, etc.). Two of the most commonly used principles for measuring intraocular pressure are the measurement of the force required to applanate a certain area of the surface of the eye, or the measurement of the diameter of the area that is applanated by a known force. These methods require the patient's co-operation and cannot be applied without general anaesthesia to small children, persons suffering from dementia, or animals.

Methods, such as those presented in the US patent publications, 5148807, 5279300, and 5299573, in which the surface of the cornea is not touched, the intraocular pressure being measured instead with the aid of a water or air jet, or various kinds of waves, have also been developed. These methods are technically complex and thus expensive. Meters operating on the air-jet principle are widely used by opticians, but their cost has prevented them from being more extensively used by general practitioners.

US patent publication 5176139 discloses a method, in which a freely-falling ball is dropped onto the eyelid and the height of the ball's rebound is measured.

The Finnish patent 109269 presents an apparatus, which is based on the fact that a probe is forwarded with a certain speed to contact the surface of the eye, wherefrom it will rebound. The movement of the probe can be a basis for the calculation of the intraocular pressure. Such apparatuses are also presented in WO publication 03/105680, US patent application 200510137473 and US patent 6,093,147 of the applicant.

US patent 6,093,147 comprises such an apparatus for measuring intraocular pressure. The apparatus comprises a probe, which is propelled at a constant velocity to impact the eye and includes means for continuously determining the velocity of the probe. The velocity is used to derive the intraocular pressure. The probe is propelled by means of a coil and a permanent magnet, whereby a current flowing through the coil causes a repelling force in the magnet. A similar device is presented in US patent application 2005/0137473 and WO publication 03/105680.

The problem in connection with many of these last mentioned apparatuses of prior art is, however, that the speed of the probe is not high enough for proper measurements.

Publication US 2005/0137474 A1 presents a method for measuring intraocular pressure.

### OBJECT OF THE INVENTION

The object of the invention is therefore primarily to achieve a sufficient speed for the probe in an apparatus, which is based on the fact that a probe is forwarded with a certain speed to contact the surface of the eye, wherefrom it will rebound.

### SUMMARY OF THE INVENTION

The method of the invention is used for measuring intraocular pressure by means of an apparatus, which includes a probe supported in a case, one coil around the probe for giving the probe a specific velocity in order to bring it in contact with the surface of the eye, and another coil for performing measurement and setting functions, means for processing and displaying the measurement data and means for controlling operations. The probe is formed of a non-magnetic front part and a back part. The method is mainly characterized by inducing magnetization into the back part of the probe by means of one or more coils before the probe is put into movement against the eye for the measurement.

Different embodiments of the invention are presented in the subclaims.

It is important that the speed of the probe when it is forwarded towards the eye is high enough for proper measurements. For this purpose, the magnetic properties of the back part of the probe have to be appropriate, i.e. the magnetization level of the magnetic part of the probe has to be high enough.

In the invention, a magnetization or a strengthening of the magnetization can be performed in several steps until a sufficient magnetization level for the probe is achieved. The measurement of the intraocular pressure itself then takes place before the magnetization has disappeared, usually it is performed shortly thereafter. In some embodiments, the time until the measurement has to be performed after the magnetization step is known and tested or calculated in advance. In practice, however, the magnetization is easy to carry out by means of the invention, and the performing of the magnetization is not a problem and being done usually before each measurement. In this way, the invention solves the problem of insufficient speeds of the probe due to low magnetization of the probe and each measurement gives a more accurate result than the methods of prior art.

The apparatus used in the invention is connected to a case component of a suitable material inside which all the components essential for the measurement are fitted. An adjustable support is needed in order to adjust the distance from which a probe impacts the eye being measured. The result of the measurement can be shown on a display and control component and an operating switch, when pressured, releases the probe towards the eye.

The method of the invention is simple, economical, and precise and the intraocular pressure can be measured in patients incapable of co-operating, and who can be restrained only momentarily.

In the following the invention is presented in detail by means of an example to which the invention is not restricted.

### FIGURE

Figure 1 is a general view of an embodiment of the apparatus of the invention

Figure 2 shows a vertical cross-section of the above, rotated to an angle of 90 degrees to the above

Figure 3 is a more detailed view of the part of figure 1, in which the probe is launched towards the eye.

### DETAILED DESCRIPTION

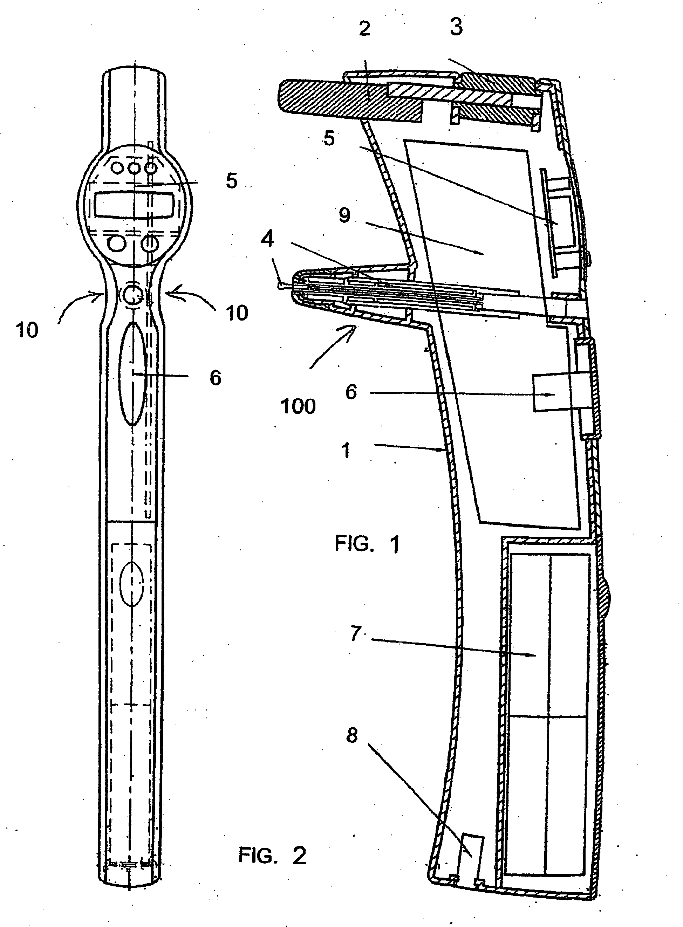

Thus, figure 1 presents, as stated above, one practical embodiment of an apparatus applying the invention, while figure 2 shows a cross-section of it. These two figures can be used together to illustrate the general principle and construction.

The apparatus is formed of a case component 1 made of a suitable material, inside of which all the components essential for the measurement are fitted.

In this embodiment, the case or body component 1 is essentially elongated and includes at its upper end a forehead support 2, which is intended to adjust the distance from which the probe 4 impacts the eye being measured. The forehead support 2 is specifically adjustable, e.g. by means of a wheel 3, which can be rotated manually.

The apparatus further includes a display and control component 5, which is e.g. a liquid-crystal panel, in which the measurement result is displayed and related control buttons etc. Reference number 6 is the operating switch, which when pressed, releases the probe 3 towards the eye.

The operating power can be taken from dry cells or batteries 7, while the apparatus additionally can have a socket 8, to which an external recharging device or power supply can be connected. The narrowings 10 make the apparatus comfortable to use.

The electronics of the apparatus are assembled on a circuit board.

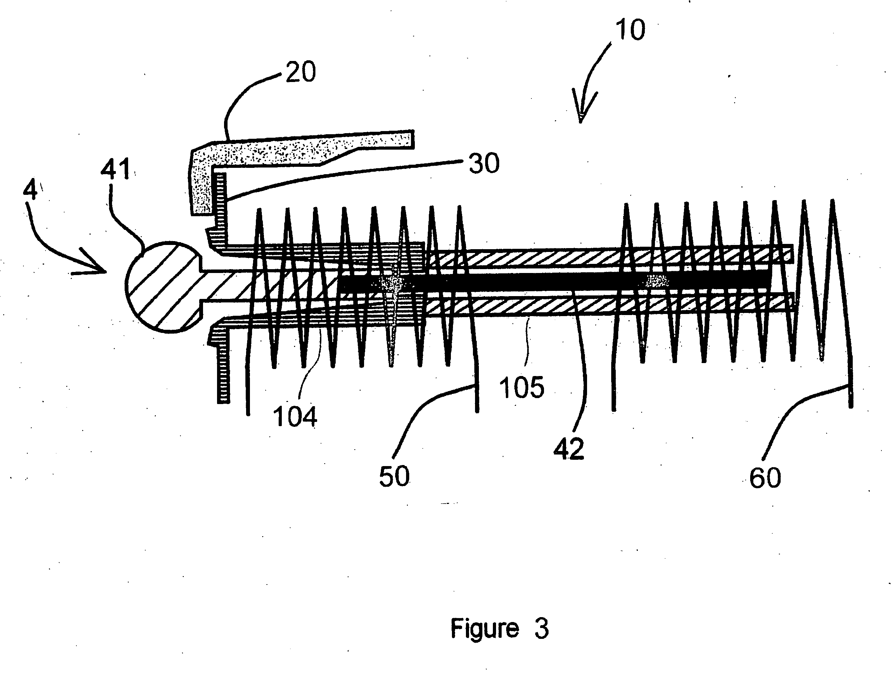

Figure 3 shows the part related to the launch of the probe 4, the recording of the measurement and other operations.

The probe 4 is supported and located in the case 1 (shown in figure 1). Figure 3 shows two coils 50, 60 of the solenoid type around the probe 4, the front one of which is intended to provide the probe 4 with a specific velocity for impacting against the eye when the intra ocular pressure of the eye is measured and being oriented accordingly. In figure 3, the back coil 60 is to provides a movement of the probe 4 in an opposite direction when activated with a voltage.

Different embodiments are possible with respect to the arrangement of the coils and the direction of the applied voltage in order to achieve desired movement directions for the probe. In the arrangement, the (mutual) orientation and distance of the coil(s), the number and size of the coils, the positions of the coils with respect to part 42, the length of the probe, and the direction of the current caused by the applied voltage can be varied.

When the ends of the back part 42 of the probe are inside the respective two coils a sin figure 3 and both coils are directed in the same direction, the coils provides opposite directions for the movement of the probe since one of the coils are around the positive pole of the magnetic back part and the other coil around the negative pole.

In one embodiment the orientation of the two coils are the same, giving the probe a movement in opposite directions since

An inner tube 105 is held in place by an inner sleeve 104, which lies around the probe and is secured by means of a separate plug 103 particularly equipped with a screw attachment. The inner tube 105 and the inner sleeve 104 can be changed, because extraneous material collected over time might disturb the measurement.

In this embodiment, the probe 4 is formed of a non-magnetic front part 41 and a magnetic or a magnetizable rear shaft part 42. The material of the shaft part 42 is chosen from paramagnetic or ferromagnetic materials. Steel is an example of a ferromagnetic material. The front part is preferably of plastic of technical and cost reasons. There is, preferably a shoulder, wherein the front part joins the shaft and is made to rest on the edges of the opening of the inner sleeve 104, through which the shaft 42 runs.

The front part 41 is in such a ratio to the magnetic shaft that the shaft extends for a certain distance inside the coil 50, e.g. half into the coil, counting from its front.

A voltage fed to the front coil 50 induces a pushing force in the probe, causing it to move towards the eye (actually in the direction where the eye is intended to be).

The two coil arrangement of figure 3 is used as an example, in which one of the coils 50 (the front coil in figure 3) is used for moving the probe in one direction while the other coil 60 (the rear coil in figure 3) is used to measure the movement of the probe 4. After the measurement, the measuring coil 60 is also used for moving the probe 4 in the opposite direction back to the original position for the next measurement or it is changed to another probe 4. Another number of coils could, however, be used as well. For example, an arrangement with three coils could have one coil for moving the probe forward, another coil for measuring the movement of the probe and a third coil for moving it backwards. Also the orientation of the coils could be varied in different embodiments, as was already mentioned before. E.g. the orientations of the coils in figure 3 could be the opposite and consequently, the operations mentioned above and below would be vice versa.

The probe is formed of a front part 41 of a non-magnetic material such as e.g. plastics and of a back part 42, which is of a paramagnetic or ferromagnetic material. The joint between parts 41 and 42 is located preferably somewhere inside the front coil 50, e.g. approximately in the middle of the coil.

The two parts 41 and 42 of the probe can also be separate pieces, so that the front part 41 is disposable (for example, plastic) and the back part 42 is located inside the device and acts like a holder for the front part. In such an arrangement, the back part is kept from falling out of the device by some internal mechanical construction. The part can be changed by opening part 20.

Part 20 is a plug or a nut by the aid of which the above described components are fastened to the case of the apparatus. An inner sleeve 30 can be replaceable after e.g. the measurement or several measurements have been performed.

It is important that the speed of the probe when it is forwarded towards the eye is high enough for proper measurements. For this purpose, the magnetic properties of the back part 42 of the probe 4 have to be appropriate, i.e. the magnetization level of the magnetic part of the probe has to be high enough.

Thus, according to the method of the invention the applicants have discovered that the coils, in addition to being used for moving the probe for the measurements, also can be used for magnetization of the back part of the probe 4 and also for strengthening the magnetization of the back part, when it is made of a paramagnetic material and/or it already has been magnetized in some extent in earlier magnetization steps.

Several embodiments are possible to perform the invention, e.g. only one of the coils can be used for the magnetization (not forming part of the present invention) or two or more coils can be used for the magnetization.

When only one coil is used for the magnetization, it is preferably the one causing a movement for the probe backwards from the eye in order to hinder the probe to move forward. It could be possible to use the other coil instead but then the movement of the probe forwards should preferably be prevented during the magnetization (such as with some holding mechanism) or by means of an oppositely directed coil during the magnetization or very shortly after the same. Otherwise the coil would cause an unnecessary movement of the probe and it could e.g. fall out from the device.

In such an embodiment, wherein both the coils are used for the magnetization, a DC voltage is led to the front coil 5 as well as to the back coil 6 at least once for a short period before the probe 4 is put into movement against the eye. In this way the magnetization of the part 42 of the probe gets the highest magnetization possible. The direction of the magnetic field caused by the current in both coils 5 and 6 is such that they strengthen their mutual effect on magnetization. A microprocessor is used to control the switches feeding the voltage to the coils.

The voltage supplied depends of different factors, such as the material of the probe.

According to the invention, the magnetization phase might be effected several times before the probe 4 is shot towards the eye. When both coils are activated, the probe 4 does not move away from its original position. From outside, a certain trembling of the probe head can possibly be observed since when applying voltage for a short period to the front coil (in figure 3 the one moving the probe towards the eye) it actually moves a very short distance forwards but is quickly moving backwards again when the voltage is directed to the back coil instead.

Thus, magnetization of the back part 42 is performed by switching a short duration DC voltage into the coil(s) 50 and/or 60 at least once in turns by switching fast enough so as to hinder the probe 4 to move forwards when magnetizing coil 50. The duration of each voltage feeding is e.g. in the order of 10 - 50 ms. In a certain embodiment the magnetization of the back part 42 is performed by switching a short duration DC voltage into the coils 50, 60 as several consecutive steps in turns.

When the magnetization is ready and the probe is wanted to be put into movement towards the eye, which should take place before the magnetization level has decreased too much, voltage is fed only to the front coil 50 thus avoiding the holding effect of the back coil 60. As the probe has a sufficiently high magnetization level, the speed of the probe is steady and enough for any measurement.

The apparatus of the invention is constructed in such a way that when it is switched on, magnetization of the probe 4 takes place automatically. During the automatic magnetization, one of the coils can be used for measuring the movement of the probe and thus used for automatic detection of the presence of the probe (or the magnetization level). If the probe is not present, the device instructs the user to load the probe.

Thus, the operation of the whole apparatus after magnetization takes place (simplified for illustrative purposes) so that power (a voltage) is supplied to the front coil 50 causing the probe to start moving and to impact the eye. When the probe 4 impacts the eye, the motion of the probe 4 is changed. The probe 4 rebounds as a result of contact with the eye and the movement backwards, takes place in a manner depending on the intraocular pressure. The movement of the probe is recorded by means of the rear coil 60 and processed by the same microprocessor as was used to control the switches feeding the voltage to the coils. The result is displayed by the display device 5. In practice, the rear coil detects the speed of the probe continuously. The measurement results of the continuous motion of the probe are used to derive the intraocular pressure. The intraocular pressure is calculated from the motion parameters of the probe.

The rear coil can be activated immediately after rebound from the eye for moving the probe back to the starting position.

As has clearly been demonstrated by the above, the method according to the invention has properties, by means of which ease of use, accuracy, and reliability can be improved and maintained. Many variations are possible while remaining within the scope of the inventive idea and the accompanying claims.

## Claims

1. A method for measuring intraocular pressure by means of an apparatus, which includes a probe (4) supported in a case, a front coil (50) around the probe for giving the probe (4) a specific velocity in order to bring it in contact with the surface of the eye, and a back coil (60) for performing measurement and setting functions, means for processing and displaying the measurement data and means for controlling operations, whereby the probe (4) is formed of a non-magnetic front part (41) and a back part (42),
**characterized in that**
the method includes a step for inducing magnetization into the back part (42) of the probe before the probe (4) is put into movement against the eye for the measurement by means of both the front coil (50) and the back coil (60), wherein the front coil (50) being closer to the eye during measurement than the back coil (60).

2. A method according to claim 1, **characterized in that** the front coils (50,) and the back coil (60) are oriented with respect to each_other in order to achieve opposite directions for the movement of the probe (4).

3. A method according to claim 2, **characterized in that** the front coil (50) being closer to the eye during measurement is oriented to give the probe (4) a forward movement towards the eye and the back coil (60) is oriented oppositely to give the probe (4) a backward movement from the eye.

4. A method of claim 1, **characterized in that** the magnetization of the back part (42) is performed by switching a short duration DC voltage into the coils (50, 60) at least once in turns by switching fast enough so as to some extent prevent the probe (4) to move forwards when magnetizing the front coil front (50) that provides a forward movement of the probe (4).

5. A method of claim 4, **characterized in that** the magnetization of the back part (42) is performed by switching a short duration DC voltage into the coils (50, 60) as several consecutive steps in turns.

6. A method of any of claims 1 - 5, **characterized in that** the number of magnetization steps is determined on the basis of the measurement result of the DC voltage performed by the back coil (60).

7. A method of any of claims 1 - 6, **characterized in that** the material of the back part (42) of the probe is chosen from paramagnetic or ferromagnetic materials.

8. A method of any of claims 1, 4, 5 and 6, 7 if not dependent on 2 or 3, **characterized by** using the front coil for giving the probe a forward movement towards the eye, the back coil for measuring the back movement of the probe and a third coil for giving the probe a backward movement from the eye.

## Patentansprüche

1. Verfahren zum Messen von intraokularem Druck mit Hilfe einer Vorrichtung, die eine Sonde (4) enthält, die in einem Gehäuse gehalten wird, sowie eine vordere Spule (50) um die Sonde herum, die der Sonde (4) eine bestimmte Geschwindigkeit verleiht, um sie mit der Oberfläche des Auges in Kontakt zu bringen, und eine hintere Spule (60) zum Ausführen von Mess- und Einstellungsfunktionen, Mittel zum Verarbeiten und Anzeigen der Messdaten und Mittel zum Steuern des Betriebs, wobei die Sonde (4) aus einem nicht magnetischen Vorderteil (41) und einem Hinterteil (42) gebildet ist,
**dadurch gekennzeichnet, dass**
das Verfahren einen Schritt zum Induzieren einer Magnetisierung in dem Hinterteil (42) der Sonde enthält, bevor die Sonde (4) in eine Bewegung gegen das Auge zur Messung versetzt wird, mit Hilfe sowohl der vorderen Spule (50) wie auch der hinteren Spule (60), wobei die vordere Spule (50) während der Messung dem Auge näher ist als die hintere Spule (60).

2. Verfahren nach Anspruch 1, **dadurch gekennzeichnet, dass** die vordere Spule (50) und die hintere Spule (60) in Bezug zueinander ausgerichtet sind, um entgegen gesetzte Richtungen für die Bewegung der Sonde (4) zu erreichen.

3. Verfahren nach Anspruch 2, **dadurch gekennzeichnet, dass** die vordere Spule (50), die während der Messung dem Auge näher ist, so ausgerichtet ist, dass sie die Sonde (4) in eine Vorwärtsbewegung zu dem Auge versetzt, und die hintere Spule (60) entgegen gesetzt ausgerichtet ist, um die Sonde (4) in eine Rückwärtsbewegung von dem Auge weg zu versetzen.

4. Verfahren nach Anspruch 1, **dadurch gekennzeichnet, dass** die Magnetisierung des Hinterteils (42) durchgeführt wird, indem eine kurzfristige Gleichspannung in die Spulen (50, 60) mindestens jeweils einmal geschaltet wird, wobei rasch genug geschaltet wird, so dass bis zu einem gewissen Maß verhindert wird, dass sich die Sonde (4) vorwärts bewegt, wenn die vordere Spule (50) magnetisiert wird, die für eine Vorwärtsbewegung der Sonde (4) sorgt.

5. Verfahren nach Anspruch 4, **dadurch gekennzeichnet, dass** die Magnetisierung des Hinterteils (42) durchgeführt wird, indem eine kurzfristige Gleichspannung in die Spulen (50, 60) in jeweils mehreren aufeinander folgenden Schritten geschaltet wird.

6. Verfahren nach einem der Ansprüche 1 bis 5, **dadurch gekennzeichnet, dass** die Anzahl von Magnetisierungsschritten auf der Basis des Messergebnisses der Gleichspannung bestimmt wird, das von der hinteren Spule (60) erhalten wird.

7. Verfahren nach einem der Ansprüche 1 bis 6, **dadurch gekennzeichnet, dass** das Material des Hinterteils (42) der Sonde aus paramagnetischen und ferromagnetischen Materialien ausgewählt ist.

8. Verfahren nach einem der Ansprüche 1, 4, 5 und 6, 7, wenn nicht abhängig von 2 oder 3, **gekennzeichnet durch** die Verwendung der vorderen Spule, um die Sonde in eine Vorwärtsbewegung zu dem Auge hin zu versetzen, der hinteren Spule zum Messen der Bewegung der Sonde und einer dritten Spule, um die Sonde in eine Rückwärtsbewegung von dem Auge weg zu versetzen.

## Revendications

1. Procédé pour mesurer la pression intraoculaire au moyen d'un appareil, qui comprend une sonde (4) supportée dans un boîtier, une bobine avant (50) autour de la sonde pour donner à la sonde (4) une vitesse spécifique afin de l'amener en contact avec la surface de l'oeil, et une bobine arrière (60) pour effectuer des fonctions de mesure et de réglage, des moyens pour traiter et afficher les données de mesure et des moyens pour commander les opérations, moyennant quoi la sonde (4) est constituée d'une partie avant (41) non magnétique et d'une partie arrière (42),
**caractérisé en ce que**
le procédé comprend une étape pour induire une magnétisation dans la partie arrière (42) de la sonde avant que la sonde (4) ne soit mise en mouvement contre l'oeil pour la mesure au moyen à la fois de la bobine avant (50) et de la bobine arrière (60), la bobine avant (50) étant plus près de l'oeil pendant la mesure que la bobine arrière (60).

2. Procédé selon la revendication 1, **caractérisé en ce que** la bobine avant (50) et la bobine arrière (60) sont orientées l'une par rapport à l'autre afin d'obtenir des directions opposées pour le mouvement de la sonde (4).

3. Procédé selon la revendication 2, **caractérisé en ce que** la bobine avant (50) qui est plus près de l'oeil pendant la mesure est orientée pour communiquer à la sonde (4) un mouvement vers l'avant vers l'oeil et la bobine arrière (60) est orientée à l'opposé pour communiquer à la sonde (4) un mouvement vers l'arrière à partir de l'oeil.

4. Procédé selon la revendication 1, **caractérisé en ce que** la magnétisation de la partie arrière (42) est effectuée en commutant une tension continue de courte durée dans les bobines (50, 60) au moins une fois chacune à son tour en commutant suffisamment rapidement pour empêcher dans une certaine mesure que la sonde (4) ne se déplace vers l'avant lors de la magnétisation de la bobine avant (50) qui permet un mouvement vers l'avant de la sonde (4).

5. Procédé selon la revendication 4, **caractérisé en ce que** la magnétisation de la partie arrière (42) est effectuée en commutant une tension continue de courte durée dans les bobines (50, 60) en tant que multiples étapes consécutives chacune à son tour.

6. Procédé selon l'une quelconque des revendications 1 à 5, **caractérisé en ce que** le nombre d'étapes de magnétisation est déterminé sur la base du résultat de la mesure de la tension continue effectuée par la bobine arrière (60).

7. Procédé selon l'une quelconque des revendications 1 à 6, **caractérisé en ce que** le matériau de la partie arrière (42) de la sonde est choisi parmi des matériaux paramagnétiques ou ferromagnétiques.

8. Procédé selon l'une quelconque des revendications 1, 4, 5 et 6, 7 si elles ne dépendent pas des revendications 2 ou 3, **caractérisé par** l'utilisation de la bobine avant pour communiquer à la sonde un mouvement vers l'avant vers l'oeil, de la bobine arrière pour mesurer le mouvement de la sonde et d'une troisième bobine pour communiquer à la sonde un mouvement vers l'arrière à partir de l'oeil.
